# EUROPEAN PATENT APPLICATION

(11) **EP 0 792 659 A2**
(43) Date of publication of application: **03.09.1997**
(21) Application number: 97300945.9
(22) Date of filing: 13.02.1997
(51) Int. Cl.: A61M 25/06

(54) **Introducer needle assembly with orienting grip**

(30) Priority: 29.02.1996 US 609091
(71) Applicant: Becton Dickinson and Company, Franklin Lakes, New Jersey 07417-1880 (US)
(72) Inventor: Musgrave, Kenneth C., Sandy, Utah, 84092 (US); Erskine, Timothy J., Sandy, Utah 84092 (US)
(74) Representative: Ruffles, Graham Keith

(57) **Abstract**

The needle assembly of this invention includes a pair of fingergrips connected to opposite sides of the main body portion of the needle hub. Each fingergrip has a generally elliptical facing surface, a flat bottom and is arcuate over a major portion thereof and is located asymmetrically to the needle hub . In addition, raised bumps are located on each facing surface for better gripping of the assembly. The fingergrips are affixed to the main body portion of the needle hub with a particular orientation to the needle bevel so that the clinician will intuitively know what the orientation of the needle bevel is when the fingergrips are grasped.

## Description

### Background of the Invention

This invention relates to an introducer needle assembly having an orienting grip to facilitate proper use of the device by a clinician.

Introducer needles are used to gain access to the venous system of a patient to permit proper placement of an intravenous (IV) catheter into a patient. Such introducer needles include a sharp distal tip and a proximal end. A hub is connected to the proximal end of the introducer needle. The hub typically includes a flashback chamber that is a reservoir to hold blood that flows from the sharp distal tip, through the needle and then out of the proximal end of the needle when the sharp distal tip of the needle pierces the patient's vein.

IV catheters are typically located coaxially over the introducer needle in a snug fit. The distal end of the needle protrudes past the distal end of the catheter. In this way, the catheter rides with the needle through the patient's skin and tissue into the vein. Once flashback of blood is observed by the clinician, the catheter is advanced further into the vein and the needle is removed from the catheter and patient.

Clinicians that insert IV catheters into a patient have a certain technique. Typically clinicians prefer to insert the introducer needle into the patient with the bevel, or the opening into the needle lumen, on the distal tip of the needle facing up away from the patient's skin. This bevel position is preferred because it allows blood to easily flow into the needle as soon as the vein has been punctured by the needle. If the needle bevel were oriented down toward the patient's skin, the opening to the needle lumen would be occluded by the vein wall until the distal tip of the needle is inserted a substantial distance into the patient's vein. This could result in the clinician inserting the needle through the opposite wall of the vein because of the delay in flashback being transmitted to the flashback chamber. In addition, having the bevel in the "up" position allows the clinician to more easily locate where the distal tip of the needle is entering the patient's skin.

Unfortunately, typical introducer needles do not have any mechanism for the clinician to know in which direction the bevel is facing. Instead, the clinician must look closely at the distal tip of the needle to know what the orientation of the bevel is. In addition, the needle hub of typical introducer needles is cylindrical. Thus even when the clinician grasps the needle hub so the bevel is in the correct orientation, the cylindrical needle hub could be rotated by the clinician so the bevel moves to an incorrect orientation during insertion.

### Summary of the Invention

It is therefore an object of this invention to provide an introducer needle assembly that facilitates use thereof with the needle bevel in the proper orientation without the need for the clinician to actually observe the needle bevel.

It is another object of this invention to provide an introducer needle assembly that allows the clinician to maintain the needle bevel in the proper orientation during insertion into a patient.

The introducer needle assembly of this invention includes a hub connected to the proximal end of the needle. The hub includes two over-sized fingergrips that are located on opposite sides thereof. The proximal end of the needle is connected to the hub so that a plane defined by a line extending between the distal end of the bevel and the proximal end of the bevel and the longitudinal axis is substantially parallel to the height of the fingergrips. Although this parallel relationship is preferred, the height of each of the fingergrips could be up to about 30 degrees away from being parallel to the plane. In this manner, when the clinician grasps the fingergrips, the clinician will immediately know in what orientation the bevel is. In addition, each fingergrip has a generally arcuate facing surface and a plurality of raised bumps formed on the facing surface to make it easier for the clinician comfortably to hold the fingergrips.

### Brief Description of the Drawings

The above and other objects and advantages of this invention will be apparent from the detailed description and drawings in which like parts are referred to by like numbers throughout, and in which :
FIG. 1 is a perspective view of the introducer needle assembly of this invention with a catheter introducer coaxially located thereover;
FIG. 2 is a perspective view of the introducer needle assembly of this invention;
FIG. 3 is a top plan view of the introducer needle assembly of this invention.
FIG. 4 is a side elevation view of the introducer needle assembly of this invention; and
FIG. 5 is a proximal end elevation view of the introducer needle assembly of this invention without the flow control plug.

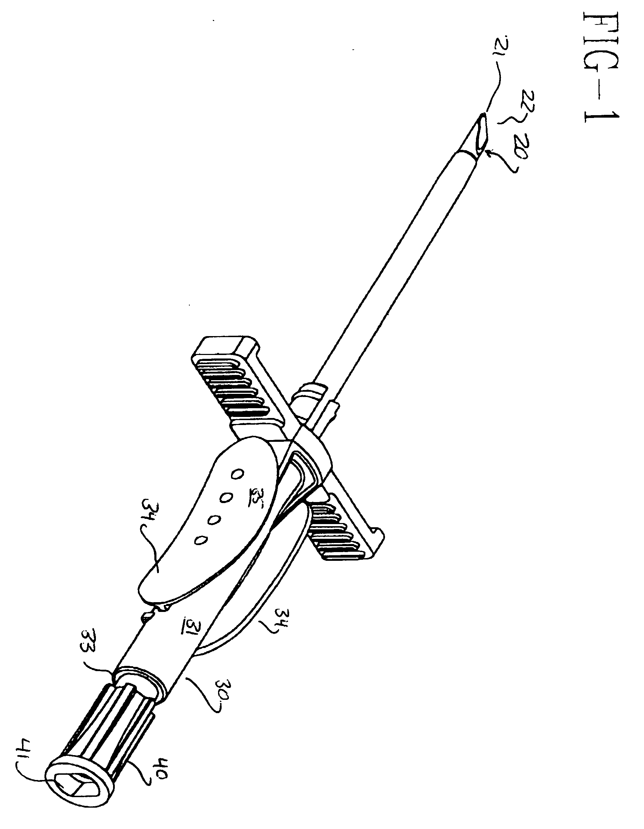

### Detailed Description of the Invention

Although this invention is described as being an introducer needle assembly, it is to be understood that this invention is applicable to other types of needle assemblies such as spinal needles, guidewire introducer needles, through the needle introducers and needle assemblies for catheter introducers.

Introducer needle assembly 10 includes a needle 20 and a needle hub 30 and a flow control plug 40 which all have a longitudinal axis A-A.

Needle 20 includes a sharp distal tip 21 and a proximal end 29. Sharp distal tip 21 is in the form of a bevel 22 which includes a distal end 22a and a proximal end 22b. Proximal end 29 of needle is affixed to needle hub 30 by any standard mechanism such as by using an adhesive. Preferably a UV cured epoxy is used to bond needle 20 to needle hub 30.

Needle hub 30 is formed from any strong, transparent polymer such as propionate. The main body portion 31 of needle hub 30 has a generally tubular configuration and is hollow. This hollow interior defines the flashback chamber 32. In order to prevent blood from leaking out of the proximal end 33 of needle hub 30, flow control plug 40 is located in open proximal end 33. Flow control plug 40 may be formed from a porous low density polyethylene such as the material sold under the POREX trademark by Porex Materials Corporation. Alternatively, flow control plug 40 can be a plastic plug that fits into open proximal end 33 and has a hydrophobic membrane or any material that is impervious to blood but will allow air to flow through it placed over the open proximal end 41 of flow control plug 40. A hydrophobic membrane or other thin porous film material could be placed directly over open proximal end 33.

Located on opposite sides of main body portion 31 are a pair of fingergrips 34. Each fingergrip 34 is connected to main body portion 31 by any standard mechanism such as by using an adhesive or by ultrasonic welding. Alternatively, fingergrips 34 could be molded integrally with main body portion 31.

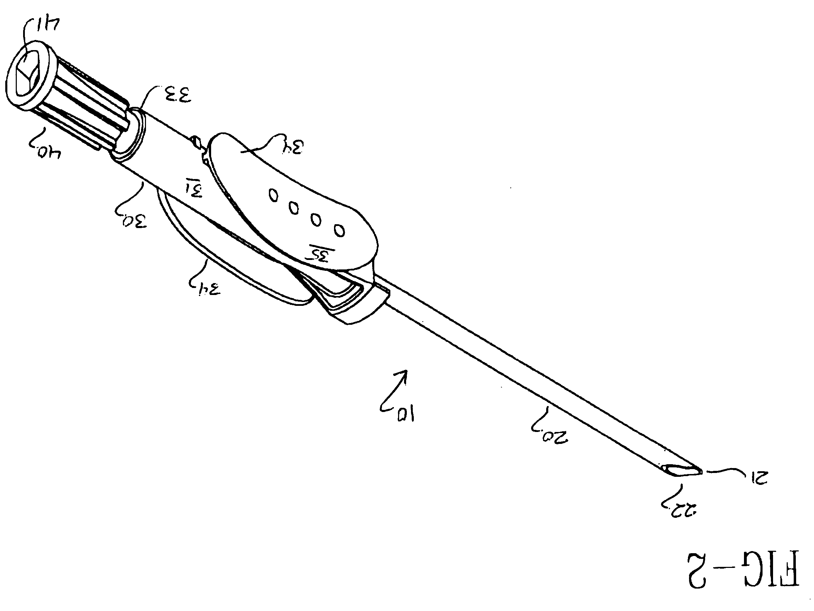

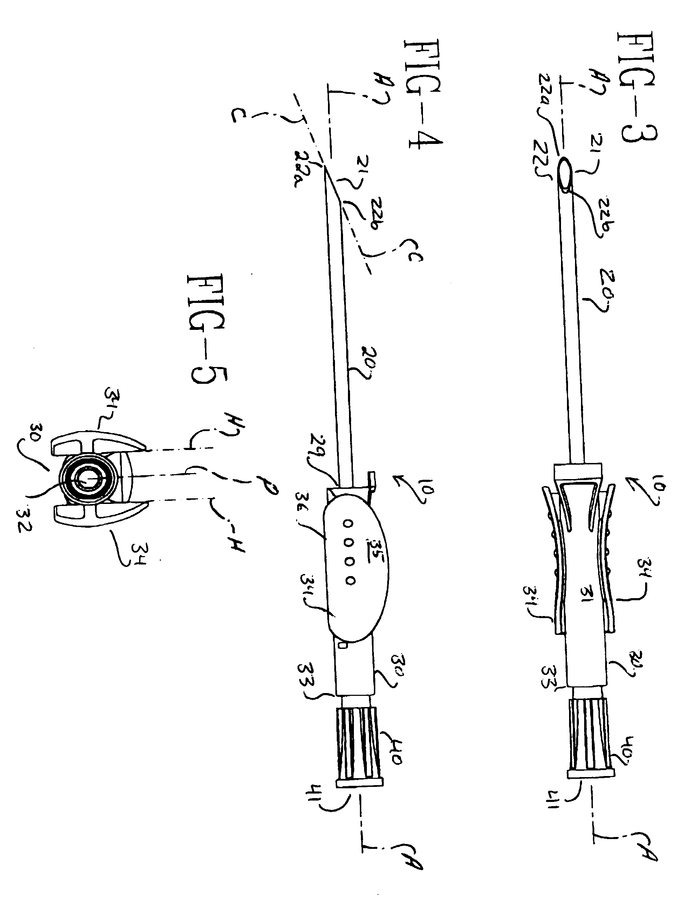

Each fingergrip 34 is connected to main body portion 31 so that each fingergrip 34 is about 180° apart from one another. In addition, each fingergrip 34 defines a height H, see FIG. 5, that is substantially parallel to the height H of the other fingergrip 34. Preferably the center of gravity of each fingergrip 34 is above longitudinal axis A-A of main body portion 31. As used herein, the terms "above" and "up" refer to the direction toward the top of FIG. 4 when the sheet is in the landscape orientation. This asymmetrical orientation of fingergrips 34 facilitates gripping of fingergrips 34 by the clinician. In addition, this configuration allows the clinician to insert introducer needle assembly 10 into a patient with a lower angle of attack. This facilitates successful insertion of introducer needle assembly 10 in the vein.

Each fingergrip 34 has a generally elliptically shaped facing surface 35 where the bottom portion has been removed to create a flat bottom 36. The ellipse forming fingergrips 34 should have a height to width ratio of between about 1:1 to about 1:4. Preferably the ellipse forming fingergrips 34 has a major axis, i.e. width, of about 1 inch and a minor axis, i.e. height, of about 0.465 inches. Preferably the minor axis is about 0.376 inches when flat bottom 36 is formed on each fingergrip 34. The major axis of each fingergrip 34 is the centerline of the ellipse before flat bottom 36 is formed. Thus, after flat bottom 36 is formed on each fingergrip 34, the center of gravity of each fingergrip 34 moves up away from the major axis of the ellipse forming fingergrip 34. This results in introducer needle assembly 10 having a low profile. In addition, flat bottom 36 clearly indicates to the clinician the bottom of needle introducer assembly 10. An elliptical shape for facing surface 35 is preferred because it has no corners and is aesthetically pleasing. However, other shapes such as a rectangle could be used for each facing surface 35.

As seen in FIGS. 4 and 5, needle 20 is connected to needle hub 30 so that the opening into bevel 22 of sharp distal tip 21 is facing up away from flat bottom 36 of fingergrips 34. In other words, needle 20 is affixed to needle hub 30 such that a plane P defined by a line C extending between distal end 22a of bevel 22 and proximal end 22b of bevel 22 and longitidunal axis A-A is substantially parallel to the height H of each fingergrip 34. This configuration allows the clinician intuitively to know that bevel 22 is in the proper orientation. Although plane P is preferably parallel to each height H, each height H may be at an angle of up to about 30 degrees in either direction from being parallel to plane P.

Each facing surface 35 preferably has an arcuate surface over a major portion thereof and a plurality of bumps formed thereon to facilitate gripping by the clinician. Preferably the arcuate surface of facing surface 35 defines an arc having a radius of about 1.185 inches, although a radius of between about 0.5 inches to about 3 inches would work. Alternatively, each facing surface 35 could be flat. In addition, each facing surface 35 could include other texturing thereon instead of a plurality of raised bumps.

Thus, it is seen that an introducer needle assembly is provided that facilitates use thereof with the needle bevel in the proper orientation without the need for the clinician to actually observe the needle bevel and that allows the clinician to easily maintain the needle bevel in the proper orientation during insertion into a patient.

## Claims

1. A needle assembly having a longitudinal axis, comprising:
a needle having a sharp distal tip, a proximal end and a bevel having a distal end and a proximal end;
a needle hub having a proximal end and a distal end connected to the proximal end of the needle; and
at least one fingergrip having a height and being located on the needle hub wherein the height is at an angle of up to about 30° to a plane defined by a line extending between the distal end of the bevel and the proximal end of the bevel and the longitudinal axis.

2. The needle assembly of claim 1 wherein the fingergrip defines a center of gravity that is not aligned with the longitudinal axis.

3. The needle assembly of claim 2 wherein the fingergrip has a substantially flat bottom.

4. The needle assembly of claim 1 wherein the fingergrip has a generally elliptical configuration.

5. The needle assembly of claim 4 wherein the fingergrip has a height to width ratio of between about. 1:1 to about 1:4.

6. The needle assembly of claim 1 wherein the fingergrip has an arcuate face.

7. The needle assembly of claim 6 wherein the arcuate face is an arc having a radius of between about 0.5 inches to about 3 inches.

8. The needle assembly of claim 6 wherein the fingergrip has a plurality of bumps formed the arcuate face.
